# EUROPÄISCHE PATENTSCHRIFT

(11) **EP 0 971 718 B1**
(45) Veröffentlichungstag und Bekanntmachung des Hinweises auf die Patenterteilung: **26.06.2002**
(21) Anmeldenummer: 98910692.7
(22) Anmeldetag: 17.02.1998
(51) Int. Cl.: A61K 31/70, A61K 9/00, A61K 9/16

(54) **KOMBINATIONSPRÄPARAT FÜR ORAL APPLIZIERBARE ERYTHROMYCINE**
COMBINED PREPARATION FOR ORAL APPLICATION OF ERYTHROMYCINS
PREPARATION COMBINEE POUR L'ADMINISTRATION PAR VOIE ORALE D'ERYTHROMYCINE

(30) Priorität: 21.02.1997 DE 19706979
(43) Veröffentlichungstag der Anmeldung: 19.01.2000
(73) Patentinhaber: Lindopharm Gmbh, 40721 Hilden (DE)
(72) Erfinder: POSANSKI, Ulrich, D-79110 Freiburg (DE); JECH, Judith, D-42719 Solingen (DE); SCHNEIDER, Bernd, D-58638 Iserlohn (DE)
(74) Vertreter: Neidlein, Helga
(86) Internationale Anmeldenummer: EP9800895
(87) Internationale Veröffentlichungsnummer: WO9836756

(56) Entgegenhaltungen:
- EP-A- 0 069 097
- WO-A-91/19486
- US-A- 5 084 278

## Beschreibung

Die Erfindung betrifft ein Kombinationspräparat im Sinne eines "Kit-of-parts" für die orale Verabreichung von Erythromycinen als wäßrige Suspension, enthaltend ein beschichtetes Erythromycin-Antibiotikum als Wirkstoffkomponente und eine Sirupgrundlage.

Viele der heute im Handel erhältlichen oral applizierbaren Antibiotika sind in wäßriger Suspension nur begrenzt haltbar. Sie werden in der Regel als Trockenpulver, z.B. in Form eines Granulats, erst kurz vor ihrem Gebrauch in Wasser aufgeschlämmt. Einige dieser Suspensionen sind aufgrund der geringen Stabilität des Wirkstoffes, z.B. als Folge von Hydrolyse oder Thermolyse, zusätzlich im Kühlschrank aufzubewahren. Diese Suspensionen sind nicht nur wegen ihrer unbefriedigenden Stabilität nachteilig. Es stört außerdem der bittere, fremde Geschmack, den zahlreiche Wirkstoffe aus dieser Indikationsgruppe bzw. ihre Zersetzungsprodukte aufweisen.

In der Pädiatrie sind süße Säfte (Sirupe) beliebte Darreichungsformen zur Behandlung von akuten Erkrankungen. Besonders häufig werden Antiinfektiva in Form eines Sirups verabreicht. Der Grund für die "Beliebtheit" dieser Darreichungsform liegt in der Maskierung des bitteren Geschmacks des Wirkstoffes. Ohne den Zusatz von Süßstoffen würden viele Kinder flüssige Arzneiformen überhaupt nicht akzeptieren. Für den Therapieerfolg ist jedoch die Akzeptanz des Arzneimittels durch den Patienten und damit die regelmäßige Einnahme eine wichtige Voraussetzung.

Die Geschmacksmaskierung des schlecht schmeckenden Antibiotikums durch die Sirupformulierung alleine stellt eine unbefriedigende Problemlösung dar. Das Stabilitätsproblem des Wirkstoffes in der wäßrigen Suspension bleibt weiterhin ungelöst.

Das Befilmen, Umhüllen oder Beschichten von Wirkstoffpartikeln, z.B. Wirkstoffen, welche in Form von Kristallen, Agglomeraten, Granulaten, Pellets oder Mikrotabletten vorliegen, ist in der pharmazeutischen Technologie eine übliche Methode, um die Stabilität des Wirkstoffes in einer wäßrigen Umgebung zu verbessern. Die Wahl des Beschichtungsmittels wird der therapeutischen Aufgabenstellung angepaßt. Die Beschichtung mit einem Polymer hoher Permeabilität, z.B. magensaftlöslichem Dimethylaminoethylmethacrylat-Methacrylsäureeter-Copolymerisat vom Typ Eudragit® E (Röhm Pharma), und geeigneter Schichtdicke, erlaubt die Wirkstofffreisetzung im sauren Milieu des Magensaftes. Die schnelle Freigabe des Wirkstoffes ist dann von Bedeutung, wenn infektiöse Zustände sofort nach ihrem Auftreten innerhalb kurzer Zeit zu behandeln sind und rascher Wirkungseintritt mit höheren Dosen erwünscht ist. Durch die schnelle Freigabe des Wirkstoffs im Magen kommt es zu einer ungewöhnlich raschen Anflutung im Plasma. Bereits nach 30 Minuten erreicht z.B. der Ester Erythromycinpropionat aus Erythromycinstinoprat 90 % der maximalen Plasmakonzentration. Durch die Wahl einer Beschichtung mit geringerer Permeabilität, z.B. Acrylsäureester-Methacrylsäureester-Copolymerisat vom Typ EUDRAGIT® NE, und gegebenenfalls größerer Schichtdicke, läßt sich die Wirkstoffabgabe im Magen bei entsprechender Verlängerung der Wirkungsdauer verzögern. Dieser Effekt ist erwünscht, um eine länger anhaltende Wirkung zu erzielen, z.B. bei einer einmal täglichen Gabe.

Auch beschichtete Wirkstoffpartikel sind in wäßriger Suspension nur eine begrenzte Zeit stabil. Die unerwünschte Diffusion des Wirkstoffes in die Suspensionsflüssigkeit erfolgt unmittelbar nach dem Ansetzen der Suspension. Daraus resultieren die hydrolytische Zersetzung des Wirkstoffes und die deutlich zunehmende Verschlechterung des Geschmackes während des Anwendungszeitraumes der Suspension.

Der vorliegenden Erfindung liegt die Aufgabe zugrunde, für oral applizierbare Erythromycine pharmazeutische Suspensionen (Trockensäfte) mit akzeptablen Geschmackseigenschaften und ausreichender Stabilität herzustellen.

Es wurde überraschenderweise gefunden, daß mit einem hydrophilen, polymeren Beschichtungsmittel kationischer Natur überzogene Wirkstoffpartikel und mit einer auf einen pH-Wert ≥ 7 eingestellten Sirupgrundlage die erforderlichen Stabilitätskriterien erfüllt werden, wenn man die Suspension mit dem beschichteten Wirkstoff oder der Wirkstoffkombination unmittelbar vor der ersten Verabreichung herstellt und innerhalb der vorgegebenen Therapiedauer verbraucht.

Gegenstand der Erfindung ist ein Kombinationspräparat für die orale Verabreichung von Erythromycinen als wäßrige Suspension, enthaltend
a) Erythromycin oder ein Derivat davon in Form von Partikeln, die mit einem polymeren, hydrophilen, permeablen, quellbaren und/oder magensaftlöslichen Beschichtungsmittel beschichtet sind, und
b) eine Sirupgrundlage mit einem pH-Wert von 7 bis 9 als zweite, von der ersten Komponente a) räumlich getrennte Komponente.

In diesem Kombinationspräparat besteht die Wirkstoffkomponente a) aus beschichteten Wirkstoffpartikeln, welche man mit der Komponente b), dem Suspensionsmittel, vor der Anwendung zusammengibt Die beiden Komponenten sind in zwei getrennten Behältnissen, die in einer gemeinsamen Packung im Sinne eines "Kit-of-parts" abgepackt sind. Der Inhalt der beiden Behältnisse wird unmittelbar vor der Anwendung in einem geeigneten Gefäß vereinigt. Die beiden getrennten Behältnisse können so gestaltet sein, daß diesen jeweils eine Einzeldosierung zu entnehmen ist. Alternativ kann der Inhalt der beiden Behältnisse in einem Vorratsgefäß vereinigt wreden, aus dem bis zum Ende der Therapie die abgemessene Menge an Flüssigkeit entnommen werden kann. Das Vorratsgefäß kann eines der beiden Behältnisse sein. Die auf diese Weise hergestellte Suspension zeichnet sich durch günstige Geschmacks- und Stabilitätseigenschaften aus. Nach dem Ansetzen der Suspension bleiben der angenehme Geschmack der Flüssigkeit und die Wirksamkeit des Wirkstoffes bis zum Therapieende, also in der Regel während 5 bis 10 Tagen, erhalten.

Die weiter vorn und im folgenden verwendeten Bezeichnungen und Begriffe sind im Rahmen der Beschreibung der Erfindung, sofern sie sich nicht von selbst verstehen, wie folgt definiert:

Als Erythromycin kann in dem erfindungsgemäßen Kombinationspräparat die Erythromycin-Base an sich sowie ihre Derivate eingesetzt werden. Als Derivate sind die Salze der Erythromycin-Base, Erythromycinester, wie z.B. Ethylsuccinat, Estolat, Glucoheptonat, Lactobionat, Propionat, Stearat, insbesondere das Prodrug von Erythromycinpropionat und Acetylcystein, welches unter der Bezeichnung Erythromycinstinoprat bekannt ist, zu nennen.

Die genannten Wirkstoffe, welche eine salzbildende Gruppe aufweisen, können, falls dies nicht bereits weiter vom angegeben ist, als pharmezeutisch annehmbare Salze vorliegen, z.B. Alkalimetallsalze (Natrium- oder Kaliumsalze), Cholinsalze, Mesilate, Sulfate oder Hydrochloride. Die Wirkstoffe können zusätzlich Kristallwasser enthalten. Die Wirkstoffe sind mit anderen oral applizierbaren Antibiotika kombinierbar.

Eine besonders bevorzugte Ausführungsform der Erfindung betrifft ein Kombinationspräparat, enthaltend als Suspensionsmittel eine Sirupgrundlage mit einem zwischen 7 und 9 eingestellten pH-Wert, besonders bevorzugt ein pH-Wert zwischen 7 und 8,5, und als Wirkstoffkomponente Erythromycinester, insbesondere das Prodrug von Erythromycinpropionat und Acetylcystein.

In dem Kombinationspräparat sind die genannten Wirkstoffe, bzw. deren Kombinationen, in den für die orale Verabreichung vorgeschriebenen Dosierungen vorhanden.

Ein geeignetes hydrophiles, permeables, magensaftlösliches Beschichtungsmittel besteht aus einem filmähnlichen Material, das für säurehaltige, wäßrige Systeme, wie den Mageninhalt, durchlässig und in diesen Flüssigkeiten quellbar und/oder löslich ist.

Geeignet sind z.B. hydrophile Polyacrylate mit einem mittleren Molekulargewicht von ca. 1,0 x 10⁵ bis 1,0 x 10⁶, bestehend aus Acrylsäurepolymerisaten oder Acrylsäure-Methacrylsäure-Copolymerisaten. Die Säuregruppen der Acrylsäureund/oder Methacrylsäuremonomeren sind teilweise oder vollständig durch C₁-C₄-Alkylgruppen verestert, insbesondere durch Methyl- und/oder Ethylgruppen, wobei diese Estergruppen durch hydrophile Gruppen, insbesondere Trimethylammoniumethyl bzw. Dimethylaminoethyl ersetzt sein können.

Bevorzugte Polyacrylate sind unter dem registrierten Warenzeichen EUDRAGIT® der Fa. Röhm (Darmstadt) erhältlich. Besonders bevorzugt sind EUDRAGIT®-Handelsformen für schnell zerfallende Filmüberzüge, z.B. hydrophile, quellbare, permeable Typen auf Acrylsäureester-Methacrylsäureester-Copolymerisatebasis, insbesondere Ethylacrylsäureester-Methylmethacrylsäureester-Copolymerisate, z.B. magensaftlösliche Typen wie EUDRAGIT® E. Das Auflösungsverhalten und die Quellbarkeit können durch Zusätze weiterer Polymere, wie z.B. Ethylcellulose, Polyvinylacetat, Polyvinylalkohol, Copolymerisate aus Methacrylsäure und Methacrylsäureestern (Eudragit® S/L) und Aminomethacrylat-Copolymere (Eudragit® RS/RL), in gewünschter Weise verändert werden.

Das Beschichten der Wirkstoffpartikel erfolgt in an sich bekannter Weise unter Anwendung üblicher Beschichtungsverfahren (Rowe, R.C., Int. J. Pharm. Techn. & Prod. Mfr., 3 (1) 27-32 (1982)), indem man das Beschichtungsmittel im gewünschten Mengenverhältnis in einem Lösungsmittel oder -gemisch dispergiert bzw. löst, z.B. in Ethanol, Isopropanol, Aceton, Methylenchlorid, Wasser bzw. deren Mischungen. Gegebenenfalls können Hilfsstoffe zur besseren technischen Durchführung des Beschichtungsvorganges zugesetzt werden, wie z.B. Mg-Stearat, Talk oder Siliciumdioxid (Syloid® 244 FP oder Aerosil® 200). Die Lösung oder Dispersion wird auf den in Pulverform vorliegenden Wirkstoff unter Anwendung bekannter Verfahren, wie der Sprühumhüllung in der Wirbelschicht, aufgesprüht. Die Verfahren und Geräte sind unter den Namen Aeromatic, Freund Glatt, Wurster oder Hüttlin (Kugel-Coater), sowie im Kessel unter den Bezeichnungen Accela Cota-Verfahren oder Tauchrohrverfahren bekannt (Kala, H., et al, Pharmazie, 34 (11) 755-765 (1979)). Das beschichtete Produkt wird anschließend getrocknet bzw. die Lösungsmittel entfernt.

Im Falle besonders instabiler Wirkstoffe sind Verfahrensbedingungen zu wählen, die eine Absenkung der Prozeßtemperaturen und/oder Verkürzung des Verfahrenszeiten erlauben. Dies kann z.B. durch das Arbeiten bei vermindertem atmosphärischem Druck (Luy, B., et al, Pharm. Ind., 51, 89-94 (1989)) und/oder durch die Verwendung von konditionierter Luft oder durch Stickstoff-Konditionierung erfolgen.

Zur Herstellung der Pulverform des zu beschichtenden Wirkstoffes bedient man sich der üblichen Mahlverfahren, z.B. Mahlung in Kugelmühlen, Stiftmühlen, Mörsermühlen oder Luftstrahimühlen.

Die Herstellung des Suspensionsmittels b) erfolgt in an sich bekannter Weise, welche z.B. für die Herstellung von konventionellen Sirupgrundlagen beschrieben ist (Temperli, M., et al, Pharm. Act. Helv., 39, 741 ff (1964)). Zusätzlich wird die Sirupgrundlage mit einer geeigneten Säure bzw. Base oder einem Puffersystem auf einen pH-Wert ≥ 7 eingestellt. Hierzu sind alle pharmazeutisch akzeptablen Säure, Basen oder Salze bzw. deren Kombination verwendbar, wie z.B. Benzoesäure, Zitronensäure, Weinsäure, Bernsteinsäure, Apfelsäure, Essigsäure, Kohlensäure, Salzsäure, Phosphorsäure, bzw. deren Na⁺, K⁺ oder Ca⁺⁺-Salze, Natronlauge, Glycylglycin, Glycin, Tris(hydroxymethyl)aminomethan-(HCI) und Triethanolamin-(HCI). Zusätzlich kann der Sirup viskositätsbeeinflussende Stoffe, Netzmittel, Konservierungsmittel, Antioxidantien, Farbstoffe, Geschmackskorrigentien (Aromen), Zucker und/oder Süßstoffe enthalten.

Die Sirupgrundlage ist darüber hinaus bevorzugt hyperton, was z.B. durch Verwendung von Zuckeralkoholen wie bevorzugt Sorbit erreicht werden kann.

Die Verwendung von Farbstoffen kann der Hebung des Aussehens als auch der Kennzeichnung des Präparates dienen. Zucker, welche als Sirupgrundlage dienen können, sind z.B. Glucose, hydrierte Glucose (Lycasin®), Saccharose, Xylitol, D-Xylose, Maltose, D-Glucose, Sorbit, Glycerin, Mannitol, Dulcitol, Maltitol, Lactitol, Fructose, Dextrose, Lactulose oder Lactose bzw. deren Kombinationen. Süßstoffe, welche zur weiteren Abrundung des Geschmackes enthalten sein können, sind Saccharin-Na, Dulcin, Glycyrrhizin, Ammoniumglycyrrhizinat, Neohesperidindihydrchlacon, Neohesperidin-HCI, Glycin, Steviosid, L-Asparagyl-Lphenylalaninmethylester (Aspartam®), Natriumcyclamat oder deren Kombinationen.

Der beschichtete Wirkstoff wird vor der Anwendung in die Sirupgrundlage gegeben. Die erhaltene Suspension behält den angenehmen Geschmack und die volle Wirksamkeit des Wirkstoffs bis zum Therapieende nach i.a. 5 bis 10 Tagen bei.

Nach oraler Einnahme der Suspension erfolgt im Magen Verdünnung und Verschiebung des pH-Werts auf etwa 3 bis 4. Dadurch wird die Suspension isoton, der kationische Film löst sich im sauren isotonen Milieu schnell, und der Wirkstoff wird rasch freigesetzt.

Das erfindungsgemäße Kombinationspräparat besitzt wertvolle pharmakologische Eigenschaften und ist für die Therapie von Infektionen verschiedener Genese verwendbar, für die sich der verwendete Wirkstoff bzw. die verwendete Wirkstoffkombination eignet.

Die folgenden Beispiele erläutern die Erfindung näher:

### Beispiel 1

### Herstellung des Trockensaftgranulats

120 kg Erythromycin-Stinoprat werden in eine Vakuum-Wirbelschichtapparatur mit Wurstereinsatz vorgelegt (Glatt). Der atmosphärische Druck wird in der Wirbelschichtapparatur auf 200 mbar abgesenkt und die Produkttemperatur auf ca. 25°C eingestellt. Nach dem Erreichen stabiler Betriebsbedingungen wird eine 20 Gew.-%ige Eudragit-E®-Lösung in Aceton mittels einer Einstoffdüse in das Wirbelbett eingesprüht. Nach dem Einsprühen von 120 kg Lösung wird der Druck in dem Gerät auf ca. 30 bis 50 mbar abgesenkt und die Produkttemperatur auf 30°C zur Nachtrocknung des Granulats angehoben.

Das getrocknete Granulat wird der Vakuumwirbelschichtapparatur entnommen und durch ein Sieb mit 1 mm Maschenweite egalisiert. Dem Granulat werden 288 g Aerosil® 200 zur Verbesserung der Fließeigenschaften untergemischt. Das Granulat wird anschließend in Beutel (Sachets) oder Glasflaschen abgefüllt. Die Sirupgrundlage und das Granulat werden in einen Karton verpackt und vor der Anwendung miteinander vermischt.

### Beispiel 2

### Herstellung der Siruplösung

70 kg Karion® FP werden in einem Rührkessel mit Heizmantel vorgelegt. Die Lösung wird auf 40°C erwärmt. Danach werden 3 g Saccharin-Na, 50 g Natriumdihydrogenphosphat und 950 g Dinatriumhydrogenphosphat in den Kessel gegeben und solange gerührt, bis eine klare Lösung entsteht. Nach Abkühlen und Erhalt einer klaren Lösung wird der pH-Wert mit verdünnter Natronlauge bzw. Salzsäure auf 7,8 eingestellt. Abschließend werden dem Ansatz 5 g Himbeeraroma und Benzalkoniumchlorid als Konservierungsmittel hinzugefügt und vollständig aufgelöst. Die fertige Siruplösung wird in braune Glasflaschen mit Schraubverschluß abgefüllt.

## Patentansprüche

1. Kombinationspräparat für die orale Verabreichung von Erythromycinen als wäßrige Suspension, enthaltend
a) Erythromycin oder ein Derivat davon in Form von Partikeln, die mit einem hydrophilen, polymeren, permeablen, quellbaren und magensaftlöslichen Beschichtungsmittel beschichtet sind, und
b) eine gepufferte Sirupgrundlage mit einem pH-Wert von ≧ 7 als zweite, von der ersten Komponente a) räumlich getrennte Komponente.

2. Kombinationspräparat nach Anspruch 1, **dadurch gekennzeichnet, daß** die Sirupgrundlage einen pH-Wert von 7 bis 9, bevorzugt von 7 bis 8,5 aufweist.

3. Kombinationspräparat nach Anspruch 1 oder 2, **dadurch gekennzeichnet, daß** das Beschichtungsmittel ein Acrylsäurepolymer oder ein Acrylsäure-Methacrylsäure-Copolymer ist.

4. Kombinationspräparat nach Anspruch 3, **dadurch gekennzeichnet, daß** die Säuregruppen des Acrylsäurepolymeren oder des Acrylsäure-Methacrylsäure-Copolymeren teilweise oder vollständig durch C₁-C₄-Alkylgruppen verestert sind, wobei diese Estergruppen durch hydrophile Gruppen ersetzt sein können.

5. Kombinationspräparat nach Anspruch 3, **dadurch gekennzeichnet, daß** die Säuregruppen des Acrylsäurepolymeren oder des Acrylsäure-Methacrylsäure-Copolymeren durch Methyl- und/oder Ethylgruppen verestert sind, wobei diese Estergruppen durch Trimethylammoniumethyl- und/oder Dimethylaminoethylgruppen gegebenenfalls ersetzt sind.

6. Kombinationspräparat nach den Ansprüchen 1 bis 5, **dadurch gekennzeichnet, daß** die beschichtete Wirkstoffkomponente Erythromycinester, insbesondere das Prodrug von Erythromycinpropionat und Acetylcystein ist.

7. Kombinationspräparat nach den Ansprüchen 1 bis 5, **dadurch gekennzeichnet, daß** die beschichtete Wirkstoffkomponente Erythromycinethylsuccinat, Erythromycinestolat, Erythromycinglucoheptonat, Erythromycinlactobionat, Erythromycinpropionat oder Erythromycinstearat ist.

8. Verwendung einer gepufferten Sirupgrundlage mit einem pH-Wert von ≧ 7, vorzugsweise von 7 bis 9, noch bevorzugter 7 bis 8,5 als Suspensionsmittel zur Herstellung einer wäßrigen Suspension eines Antibiotikums in Form von Partikeln, die mit einem hydrophilen, polymeren, permeablen, quellbaren und magensaftlöslichen Beschichtungsmittel beschichtet sind.

9. Kombinationspräparat nach einem der Ansprüche 1 bis 7 zur Behandlung von bakteriellen Infektionen.

## Claims

1. A combination preparation for the oral administration of erythromycins as an aqueous suspension, containing
a) erythromycin or a derivative thereof in the form of particles which are coated with a hydrophilic, polymeric, permeable, swellable coating medium which is soluble in gastric juice, and
b) a buffered syrup base with a pH of ≥ 7 as a second component which is spatially separated from the first component a).

2. A combination preparation according to claim 1, **characterised in that** the syrup base has a pH of 7 to 9, preferably 7 to 8.5.

3. A combination preparation according to claims 1 or 2, **characterised in that** the coating medium is an acrylic acid polymer or an acrylic acid-methacrylic acid copolymer.

4. A combination preparation according to claim 3, **characterised in that** the acid groups of the acrylic acid polymer or of the acrylic acid-methacrylic acid copolymer are partially or completely esterified by C₁-C₄ alkyl groups, wherein the ester groups can be replaced by hydrophilic groups.

5. A combination preparation according to claim 3, **characterised in that** the acid groups of the acrylic acid polymer or of the acrylic acid-methacrylic acid copolymer are esterified by methyl and/or ethyl groups, wherein the ester groups are optionally replaced by trimethylammoniumethyl and/or dimethylaminoethyl groups.

6. A combination preparation according to claims 1 to 5, **characterised in that** the coated active ingredient component is an erythromycin ester, particularly the prodrug of erythromycin propionate and acetylcysteine.

7. A combination preparation according to claims 1 to 5, **characterised in that** the coated active ingredient component is erythromycin ethyl succinate, erythromycin estolate, erythromycin glucoheptonate, erythromycin lactobionate, erythromycin propionate or erythromycin stearate.

8. Use of a buffered syrup base with a pH of ≥ 7, preferably from 7 to 9, most preferably from 7 to 8.5, as a suspension medium for producing an aqueous suspension of an antibiotic in the form of particles which are coated with a hydrophilic, polymeric, permeable, swellable coating medium which is soluble in gastric juice.

9. A combination preparation according to any one of claims 1 to 7 for the treatment of bacterial infections.

## Revendications

1. Préparation combinée destinée à l'administration orale d'érythromycines sous forme de suspension aqueuse, contenant :
a) de l'érythromycine ou l'un de ses dérivés sous forme de particules munies d'un revêtement hydrophile, polymérique, perméable, apte au gonflement et soluble dans le suc gastrique, et
b) une base de sirop tamponnée, ayant un pH ≥ 7, comme second composant séparé du premier composant a).

2. Préparation combinée selon la revendication 1, **caractérisée en ce que** la base de sirop a un pH compris entre 7 et 9, de préférence entre 7 et 8,5.

3. Préparation combinée selon la revendication 1 ou 2, **caractérisée en ce que** le revêtement est un polymère d'acide acrylique ou un copolymère d'acide acrylique et d'acide méthacrylique.

4. Préparation combinée selon la revendication 3, **caractérisée en ce que** les groupes acides du polymère d'acide acrylique ou du copolymère d'acide acrylique et d'acide méthacrylique sont estérifiés en partie ou en totalité par des groupes alkyles en C₁-C₄, ces groupes esters pouvant être substitués par des groupes hydrophiles.

5. Préparation combinée selon la revendication 3, **caractérisée en ce que** les groupes acides du polymère d'acide acrylique ou du copolymère d'acide acrylique et d'acide méthacrylique sont estérifiés par des groupes méthyle et/ou éthyles, les groupes esters étant éventuellement substitués par des groupes méthyles triméthylammonium et/ou des groupes diméthylamino-éthyles.

6. Préparation combinée selon les revendications 1 à 5, **caractérisée en ce que** le composant substance active revêtu est un ester d'érythromycine, en particulier la prodrogue de l'érythromycine propionate et de l'acétylcystéine.

7. Préparation combinée selon les revendications 1 à 5, **caractérisée en ce que** le composant substance active revêtu est l'érythromycine éthylsuccinate, l'érythromycine estolate, l'érythromycine glucoheptonate, l'érythromycine lactobionate, l'érythromycine propionate ou l'érythromycine stéarate.

8. Utilisation d'une base de sirop tamponnée ayant un pH ≥ 7, de préférence compris entre 7 et 9, et plus particulièrement entre 7 et 8,5 comme agent de suspension pour la préparation d'une suspension aqueuse d'un antibiotique sous forme de particules munies d'un revêtement hydrophile polymérique, perméable, apte au gonflement et soluble dans le suc gastrique.

9. Préparation combinée selon l'une des revendications 1 à 7 destinée au traitement des infections bactériennes.
